(19) Europäisches Patentamt
European Patent Office
Office européen des brevets

(11) Numéro de publication: **0 193 439 B1**

# (12) FASCICULE DE BREVET EUROPEEN

(45) Date de publication de fascicule du brevet: **16.10.91**

(51) Int. Cl.$^5$: **G01N 33/569**, G01N 33/556

(21) Numéro de dépôt: **86400232.4**

(22) Date de dépôt: **04.02.86**

Le dossier contient des informations techniques présentées postérieurement au dépôt de la demande et ne figurant pas dans le présent fascicule.

## (54) Réactif pour le dosage par hémagglutination des anticorps contre les toxines bactériennes, procédé de préparation et son application.

(30) Priorité: **05.02.85 FR 8501579**

(43) Date de publication de la demande: **03.09.86 Bulletin 86/36**

(45) Mention de la délivrance du brevet: **16.10.91 Bulletin 91/42**

(84) Etats contractants désignés: **AT BE CH DE FR GB IT LI LU NL SE**

(56) Documents cités:
**EP-A- 0 101 166**
**FR-A- 2 403 559**
**FR-A- 2 478 470**
**US-A- 4 193 982**
**US-A- 4 403 037**

(73) Titulaire: **INSTITUT PASTEUR**
**25-28, rue du Docteur Roux**
**F-75724 Paris Cédex 15(FR)**

(72) Inventeur: **Relyveld, Edgard Hans**
**3 place du Général Stéfanik**
**F-75016 Paris(FR)**

(74) Mandataire: **Phélip, Bruno et al**
**c/o Cabinet Harlé & Phélip 21, rue de La Rochefoucauld**
**F-75009 Paris(FR)**

EP 0 193 439 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un nouveau réactif, constitué d'érythrocytes couplés, permettant un dosage par hémagglutination des anticorps dirigés contre des toxines bactériennes, le procédé de préparation de ce réactif, ainsi que le coffret de réactifs nécessaires pour la mise en oeuvre du dosage et le procédé de dosage correspondant.

On sait doser les anticorps, en utilisant l'hémagglutination somme révélateur de leur présence dans un milieu biologique; on utilise pour cela des érythrocytes porteurs d'un antigène, réagissant avec les anticorps à doser; mais les érythrocytes traités sont peu stables et c'est un inconvénient majeur de ce type de procédé, par ailleurs intéressant puisqu'il n'impose pas d'utiliser un appareillage de mesure coûteux. Dans le brevet FR-A-2 331 352 est décrit un moyen pour améliorer notamment la stabilité du réactif d'hémagglutination qui consiste à traiter les érythrocytes en suspension dans un tampon par un aldéhyde aliphatique inférieur et un sel de chrome hydrosoluble avant de les mettre en contact avec l'antigène choisi; la durée de conservation est alors améliorée si l'on conserve les érythrocytes traités à 4°C dans une solution colloïdale ou à l'état lyophilisé. Dans FR-A-2 475 737, on décrit un autre moyen d'amélioration de la stabilité des hématies traitées pour servir de réactif dans des réactions d'hémagglutination, qui diminue simultanément les phénomènes d'agglutination spontanée; du sang total est soumis à l'action d'un aldéhyde, tel que le formol ou le glutaraldéhyde, on isole ensuite les hématies et les traite par un tannin avant de les mettre en contact avec l'antigène choisi pour obtenir un réactif qui sera conservé sous forme lyophilisée.

On a maintenant trouvé un nouveau réactif pour le dosage par hémagglutination des anticorps antitoxines bactériennes, particulièrement stable, très sensible, dépourvu de toute toxicité, et donc manipulable sans précautions particulières: la technique de dosage est par ailleurs simple et rapide, et applicable avec de petits échantillons de sang sur le terrain.

Selon l'invention, le réactif qui est constitué d'érythrocytes sensibilisés à l'antigène correspondant à l'antitoxine à doser, est préparé en faisant réagir sur une suspension aqueuse d'érythrocytes du glutaraldéhyde de telle sorte que ceux-ci ne soient pas hémolysés lorsqu'ils sont ensuite mis au contact de la toxine bactérienne correspondant aux anticorps à doser, puis en traitant ces érythrocytes par une certaine quantité de la toxine bactérienne en présence de glutaraldéhyde pour fixer la toxine sur les érythrocytes et simultanément en modifier la structure de telle sorte qu'elle perde son activité toxique pour l'homme ou les animaux sans dénaturation de ses sites antigéniques.

On a déjà décrit dans FR-A-2 478 470 un procédé de préparation de cellules immunogéniques résultant de la fixation sur des lymphocytes de différents agents tels qu'antigènes, hormones, toxines, par action du glutaraldéhyde sur le mélange des lymphocytes et des agents immunogéniques; dans le cas de la fixation des toxines tétanique et diphtérique, les préparations cellulaires ainsi obtenues se trouvaient détoxifiées. Toutefois, il n'était pas évident qu'un tel procédé appliqué à des cellules aussi fragiles que les érythrocytes permette d'obtenir un réactif de dosage par hémagglutination, stable, atoxique, non-agglutinant et sensible. La stabilité des hématies sensibilisées utilisées dans les dosages par hémagglutination est un réel problème et on a cité dans ce qui précède certaines des solutions précédemment trouvées; il est surprenant qu'aucun des traitements plus ou moins complexes précédemment préconisés ne soit nécessaire pour le réactif de l'invention. Notons que le document US-A- 4,403,037 concerne un dosage par hémagglutination dirigé sur des antigènes de virus. Cependant ce document décrit un procédé de traitement des érythrocytes qui est différent de celui de l'invention dans la mesure où on ajoute directement l'antigène à une suspension non traitée puis on stabilise les érythrocytes revêtus par un agent prontant tel que le glutaraldéhyde. Par ailleurs, pour que l'hémagglutination serve comme réaction de révélation dans un procédé de dosage, elle doit avoir lieu même en présence de faibles quantités d'anticorps, et sans agglutination parasite; or on ne pouvait déduire du document FR-A-2478 470 que les toxines détoxifiées fixées sur les érythrocytes seraient encore suffisamment antigéniques pour permettre un dosage d'anticorps en faible concentration dans les milieux biologiques.

Dans une forme de réalisation préférée de l'invention, le procédé d'obtention du réactif comporte une étape supplémentaire qui consiste à traiter les hématies porteuses de la toxine détoxifiée, par un composé porteur d'une fonction susceptible de réagir sur les fonctions aldéhydes libres; le blocage des groupes aldéhydes qui en résulte améliore encore la stabilité du réactif, diminue les hémagglutinations parasites dues aux réactions avec les diverses protéines présentes dans l'échantillon à doser, ce qui augmente la sensibilité du réactif, et enfin élimine tout risque de réapparition d'une activité toxique due à une réversion partielle des structures détoxifiées de la toxine bactérienne au cours de la conservation du réactif. Comme agent de blocage, on peut citer les aminoacides tels que glycocolle ou lysine, les peptones, tels que peptones de viande et l'hydrolysat de caséine, les amines primaires telles que glycine et éthanolamine.

Parmi les toxines bactériennes qui peuvent être couplées aux érythrocytes par le procédé selon l'invention, on peut citer les toxines diphtérique et tétanique; il est préférable que ces toxines soient de haute pureté pour être couplées aux hématies; on utilise par exemple la toxine diphtérique cristallisée et dialysée contre la solution aqueuse tamponnée utilisée pour la mise en suspension des cellules, pour la réaction.

On préfère utiliser des hématies nucléées, d'oiseaux comme pigeons, dindes, dont on sait que la sédimentation est plus rapide que celle des hématies de mouton, pour préparer les réactifs de l'invention. De cette façon, un dosage utilisant le procédé de l'invention peut être réalisé en 15 minutes environ, alors qu'avec les techniques mises en oeuvre actuellement, il faut attendre plusieurs heures avant d'effectuer la lecture des résultats.

Dans une forme de réalisation particulière de l'invention, on fera réagir à température ambiante pendant quelques minutes du glutaraldéhyde sur des érythrocytes d'oiseaux en suspension dans une solution tamponnée isotonique, de pH compris entre 6,5 et 8,0 environ. La solution tampon peut être une solution aqueuse de phosphate salin, dans laquelle on mettra en suspension des érythrocytes pour obtenir une concentration de 1 à 12% (V/V); le glutaraldéhyde y est ensuite introduit sous forme d'une solution aqueuse diluée pour obtenir une concentration finale de 0,001 à 0,1 M d'aldéhyde dans la solution d'érythrocytes. On peut aussi ajouter les érythrocytes à une solution aqueuse tamponnée de glutaraldéhyde, de concentration comprise entre 0,0025 M et 0,25 M, pour obtenir les mêmes concentrations finales.

A ce stade, il n'est pas nécessaire d'isoler les érythrocytes, on peut introduire la toxine bactérienne directement dans la suspension; pour cela on ajoute une solution de toxine dans le même tampon, de concentration comprise entre 4 mg et 20 mg/ml environ, à raison de 0,4 mg à 2 mg de toxine par ml d'érythrocytes à traiter. Pour réaliser le couplage et la détoxification de toute la toxine ajoutée, le mélange doit être maintenu au moins une heure de préférence, ou 2 à 3 heures, suivant les toxines à fixer, à température ambiante. Les cellules sensibilisées sont alors isolées par centrifugation et lavées par une solution aqueuse tamponnée isotonique.

Pour saturer les groupes aldéhydes libres, on met les cellules ainsi obtenues en suspension pendant au moins 15 minutes dans une solution tamponnée de l'agent de blocage choisi, de concentration 0,1 M à 1 M environ à raison d'un volume d'érythrocytes pour 7 volumes de solution. Après les lavages habituels, les érythrocytes isolés par centrifugation peuvent être remis en suspension dans une solution aqueuse tamponnée isotonique en présence d'un agent bactéricide usuel, comme l'azoture de sodium ou le merthiolate, en concentration finale de respectivement 1:5000 et 1:10000 (p/v), et conservés tels que à 4 °C pendant plus d'une année, ou à 37 °C pendant près d'un mois; on peut aussi effectuer une lyophilisation de cette suspension, toutefois on préfère ne pas lyophiliser, car on sait que si la conservation du réactif en est amélioré, ses propriétés sont fréquemment modifiées lors de cette opération et de façon non reproductible.

Les temps de contact des réactifs dans les diverses étapes du procédé sont fonction de la température à laquelle est effectuée le traitement ainsi que de la concentration des réactifs dans la solution et de leurs quantités relatives et ces divers paramètres pourront être adaptés par le spécialiste.

Les réactifs obtenus par ce procédé sont un autre objet de l'invention. Ils sont constitués de corpuscules non toxiques, ce qui est un avantage de l'invention, consistant en érythrocytes sur lesquels ont été fixés par l'intermédiaire du glutaraldéhyde des toxines bactériennes. Les fonctions aldéhydes de ces corpuscules peuvent éventuellement avoir été saturées par réaction chimique avec un agent de blocage ; dans le cas des réactifs à base de toxine diphtérique, la saturation des fonctions aldéhydes est très souhaitable.

Les réactifs de l'invention sont avantageusement utilisés pour le dosage d'anticorps dirigés contre les toxines bactériennes correspondantes, présents dans les liquides biologiques, sans que les échantillons aient été préalablement traités par dialyse, centrifugation, précipitation ou autre; une simple dilution aqueuse sera en général suffisante. C'est un des avantages de l'invention dont l'importance doit être soulignée. En effet, aucun des procédés de dosage des anticorps antitétanique ou antidiphtérique, actuellement pratiqués, ne permettent d'utiliser le sang total; aussi dans la mesure où seul du sérum peut être utilisé, on doit prélever une quantité suffisamment importante de sang, au moins quelques ml, à l'individu dont on veut déterminer le taux de protection contre la diphtérie ou le tétanos, pour permettre une séparation du sérum par centrifugation. Cette opération, bien que courante, ne peut être effectuée que par un personnel qualifié et avec un matériel coûteux. Avec les réactifs de l'invention, le prélèvement d'une seule goutte de sang suffit pour effectuer un dosage des anticorps par le procédé de dosage de l'invention.

Ce procédé de dosage consiste à mélanger différentes dilutions de l'échantillon à étudier dans une solution aqueuse tamponnée à un pH compris entre 6,5 et 8, avec une quantité déterminée d'un réactif de l'invention, puis à observer après 15 minutes à température ambiante, et de préférence après 20 minutes, pour quelles dilutions il y a hémagglutination; la concentration en anticorps de l'échantillon est alors déterminée en se référant à un étalon. Comme il a été mentionné plus haut, l'échantillon peut être du sang frais, ou conservé sur anticoagulant ou absorbé sur une matière absorbante tel qu'un papier buvard utilisé en biologie, du sérum ou encore une préparation d'anticorps provenant d'un animal immunisé. Lorsque l'on recherche si un individu a un taux suffisant d'anticorps pour être protégé contre la diphtérie ou le tétanos, on effectue une dilution préalable du sang au 1/20 environ, dans de l'eau distillée ou faiblement acide; dans ces conditions, les globules rouges lysés et les protéines du sang n'interfèrent pas.

Pour le sérum, la dilution de départ sera en général au 1/10 ou 1/20; des dilutions successives peuvent ensuite être préparées par exemple selon une progression géométrique de raison 2.

Si l'essai est effectué en introduisant la composition de réactifs et les différentes dilutions de l'échantillon dans les cupules d'une plaque en polystyrène comportant au moins 8 puits, il suffit de 10 µl d'échantillon de liquide biologique pour effectuer un dosage, ce qui est un avantage important de l'invention. Dans le cas du dosage des anticorps antidiphtériques, il est préférable d'ajouter dans le milieu du dosage une protéine, telle que la sérum albumine humaine (SAH) ou un autre composant de forte masse moléculaire, tel que la polyvinylpyrrolidone pour que la sensibilité de la méthode soit comparable à celle obtenue pour le dosage des anticorps antitétaniques; on peut introduire la sérum albumine en même temps que la composition de réactif, en mélangeant celle-ci à une solution tamponnée de SAH.

Dans ces conditions, on peut déceler de façon reproductible la présence de 0,003 à 0,0004 unités internationales antitoxiques (UIA), dans le sang total.

La composition de réactif utilisée peut être la suspension des érythrocytes sensibilisés qui a servi à leur conservation, c'est-à-dire une suspension aqueuse tamponnée à un pH compris entre 6,5 et 8,0 environ, comportant de 1% à 20% (v/v) d'érythrocytes couplés; aucun traitement préalable de la suspension de conservation n'est nécessaire et un coffret de réactifs, objet de l'invention, peut comporter cette suspension avec un certain volume de solution aqueuse tamponnée pour dilution, un sérum de référence et la mention de la concentration en anticorps minimale donnant une hémagglutination observable, c'est-à-dire la concentration pour laquelle les hématies décantent sous forme d'un réseau homogène, réparti sur toute la surface des parois et non en un culot de petit diamètre. La composition de réactifs utilisée peut aussi être préparée instantanément ou quelque temps avant l'essai par remise en suspension des érythrocytes sensibilisés lyophilisés.

A la lecture de ce qui précède, on comprend que le procédé de dosage selon l'invention est facile à mettre en oeuvre, peu coûteux, en particulier parce qu'il ne nécessite aucun appareillage de mesure compliqué, rapide, puisque la lecture de la microplaque peut être effectuée moins de 20 minutes après l'introduction de l'échantillon, qu'il peut être pratiqué en l'absence de sources de réfrigération, puisque les réactifs ne sont pas modifiés après plusieurs semaines à la température ambiante, et pratiqué un certain temps après le prélèvement de l'échantillon, puisque le titre en anticorps d'un sang total n'est pas modifié après conservation d'un échantillon de 10 µl sur une pastille de papier buvard de 2 mm de diamètre pendant une semaine à température ambiante et plusieurs semaines à 4 °C.

L'invention est illustrée par des exemples de mise en oeuvre du procédé de préparation de l'invention pour l'obtention d'érythrocytes sensibilisés à l'anatoxine tétanique et à l'anatoxine diphtérique, ainsi que par des exemples de titrage des anticorps présents dans le sang avec des réactifs de l'invention.

Exemple 1: Couplage des toxines tétanique ou diphtérique aux érythrocytes

Des globules rouges de dinde, recueillis sur Alsever sont lavés avec un tampon phosphate isotonique à pH 7,4, contenant du NaCl (PBS), puis mis en suspension dans le même tampon à raison de 3,6 ml de globules rouges pour un volume final de 100 ml de tampon phosphate (pH = 7,4). On ajoute lentement à 100 ml de cette suspension, sous agitation, 71,5 ml d'une solution de glutaraldéhyde dans le même tampon, de concentration 0,025 M. Après une minute de contact, on introduit 71,5 ml d'une solution de toxine tétanique ou diphtérique dans le même tampon, contenant 4 mg de toxine par ml. Le mélange est agité pendant une heure pour la toxine tétanique et 2 heures pour la toxine diphtérique, à température ambiante, puis les globules rouges sensibilisés sont séparés par centrifugation et lavés 3 fois avec un volume égal (243 ml) du même tampon.

On verse alors sur le culot de centrifugation un volume égal d'une solution 0,1 M de glycocolle dans le même tampon phosphate et on laisse 30 minutes sous agitation. Les globules rouges ainsi traités sont isolés par centrifugation, lavés par 3 fois avec le même volume de tampon phosphate PBS avant d'être remis en suspension dans le PBS contenant 2:10000 (p/v) d'azoture de sodium, à raison de 5 ml de globules rouges sensibilisés pour 100 ml de tampon.

Il est important d'utiliser un tampon de pH peu basique; ainsi, les résultats obtenus avec un tampon de pH = 8,65 sont négatifs.

Exemple 2: Dosage d'anticorps antidiphtériques dans un sérum humain de référence:

Le sérum de référence, contenant 1 UAI/ml, est dilué au 1/10, puis de 2 en 2, jusqu'au 1/2560ème, dans les cupules d'une microplaque contenant 50 µl de PBS.

Dans un premier essai , on introduit ensuite dans chaque cupule, 50 µl de suspension de globules rouges sensibilisés. Dans un second essai, on ajoute dans chaque cupule 50 µl d'une suspension à 1% des globules rouges sensibilisés dans du PBS contenant aussi 0,1% de sérum albumine humain. L'aspect des puits est observé après 20 minutes de contact à température ambiante, après avoir recouvert la microplaque pour éviter l'évaporation.

Les résultats obtenus figurent dans le Tableau I ci-dessous et montrent qu'en l'absence d'albumine la détermination de la dilution limite est pratiquement impossible.

Tableau I

| Réactif | Dilutions du sérum de référence | | | | | | | | |
|---|---|---|---|---|---|---|---|---|---|
| | 1/10 | 1/20 | 1/40 | 1/80 | 1/160 | 1/320 | 1/640 | 1/1280 | 1/2560 |
| Globules sensibilisés à 1% en PBS | + | + | + | + | + | + | ± | ± | ± |
| Globules sensibilisés à 1% en PBS + sérum albumine | + | + | + | + | + | + | - | - | - |

+ : hémagglutination nette

± : hémagglutination partielle (illisible)

- : absence d'hémagglutination

Exemple 3: Dosage d'anticorps antitétaniques dans le sang

On prélève le sang par piqûre au bout du doigt avec un vaccinostyle et en dilue 10 µl avec 190 µl d'eau distillée.

Dans les deux premières cupules, on introduit 50 µl de la dilution de sang au 1/20, puis 50 µl de diluant dans les cupules 2 à 8. On effectue ensuite des dilutions géométriques des cupules 3 à 11, en y introduisant à chaque fois 50 µl de la cupule précédente, sauf dans la cupule n° 11 ce qui conduit à rejeter 50 µl de la cupule n° 10.

Dans chacune des cupules on ajoute 50 µl de globules sensibilisés obtenus selon l'exemple 1, en suspension à 1% dans le tampon phosphate salin; on agite la plaque pendant une minute et laisse incuber couvert environ 15 minutes.

Une gamme préparée avec un sérum antitétanique de référence à 1 UAI/ml effectuée simultanément montre que la dilution limite de sérum donnant une hémagglutination nette (réseau homogène réparti dans toute la cupule) correspond à la dilution du sérum au 1/2560, ce qui correspond à environ 1 UAI/ml. Dans ces conditions, la dilution du sang d'un individu à la limite de la protection contre le tétanos se trouve dans la cupule n° 3 et correspond à 0,032 UAI/ml de sang.

## Revendications

1. Procédé de préparation d'un réactif non toxique pour le dosage par hémagglutination des anticorps dirigés contre des toxines bactériennes, caractérisé en ce qu'on couple à l'aide de glutaraldéhyde les érythrocytes et la toxoïde correspondant à la toxine bactérienne, en faisant réagir en milieu aqueux tampon le glutaraldéhyde sur les érythrocytes et en ajoutant ensuite directement la toxine bactérienne au milieu réactionnel.

2. Procédé de préparation d'un réactif pour le dosage par hémagglutination des anticorps dirigés contre des toxines bactériennes dans lequel le produit obtenu selon la revendication 1 est en outre traité par un agent de blocage des fonctions aldéhydes.

3. Procédé selon l'une des revendications 1 ou 2, caractérisé en ce que
a) on fait réagir du glutaraldéhyde sur une suspension d'érythrocytes dans un milieu isotonique tamponné de pH de 6,5 à 8, pendant quelques minutes à une température comprise entre 0° C et 37° C, de telle sorte que les érythrocytes sont présents à raison de 0,75 à 8% (v/v) pour une concentration en glutaraldéhyde comprise entre 0,001 M et 0,1 M.
b) on fait réagir sur les érythrocytes ainsi obtenus, en suspension isotonique tamponnée, pendant au moins une heure de la toxine en présence de glutaraldéhyde, à raison de 0,4 mg à 2 mg de toxine par ml d'érythrocytes.
c) on lave les cellules ainsi traitées pour éliminer l'excès de réactifs.

4. Procédé selon l'une des revendications 2 ou 3, caractérisé en ce que les cellules couplées sont en outre traitées après centrifugation pendant au moins 15 minutes par un volume égal d'une solution aqueuse tamponnée de glycocolle ou de lysine, de concentration finale comprise entre 0,1 M et 1 M.

5. Procédé selon l'une quelconque des revendications précédentes caractérisé en ce qu'il concerne la préparation d'un réactif pour le dosage des anticorps antitétaniques.

6. Procédé selon l'une quelconque des revendications 1 à 4, caractérisé en ce qu'il concerne la préparation d'un réactif pour le dosage des anticorps antidiphtériques.

7. Réactif non toxique pour le dosage par hémagglutination des anticorps dirigés contre des toxines bactériennes, consistant en des érythrocytes sur lesquels est couplée par l'intermédiaire de glutaraldéhyde la toxoïde correspondant à la toxine bactérienne, la structure de ladite toxine étant modifiée de telle sorte qu'elle perde son activité toxique pour l'homme ou les animaux sans dénaturation de ses sites antigéniques.

8. Réactif selon la revendication 7, caractérisé en ce qu'il est en outre stabilisé par traitement avec un agent de blocage du glutaraldéhyde,

9. Réactif selon l'une des revendications 7 ou 8, pour le dosage des anticorps antitétaniques.

10. Réactif selon l'une des revendications 7 ou 8 pour le dosage des anticorps antidiphtériques.

11. Procédé de dosage des anticorps dirigés contre des toxines bactériennes, caractérisé en ce que l'on mélange dans une solution tampon aqueuse l'échantillon à étudier à différentes dilutions, avec un réactif selon l'une quelconque des revendications 7 à 10, que l'on observe pour quelles dilutions il y a hémagglutination et que l'on détermine la concentration de l'échantillon en antitoxine en se référant à un étalon.

12. Procédé selon la revendication 11, caractérisé en ce que l'échantillon à doser est du sang total.

13. Procédé selon l'une des revendications 11 ou 12, pour le dosage des anticorps antitétaniques.

14. Procédé selon l'une des revendications 11 ou 12 pour le dosage des anticorps antidiphtériques, caractérisé en ce que l'on introduit dans le milieu de dosage de la sérum albumine humaine ou un composant analogue.

15. Coffret de réactifs pour le dosage par hémagglutination des anticorps dirigés contre des toxines bactériennes caractérisé en ce qu'il comprend un réactif selon l'une quelconque des revendications 7 à 10, sous forme d'une suspension aqueuse tamponnée comportant des erythrocytes couplés et un agent conservateur, une solution aqueuse tamponnée et un sérum de référence.

## Claims

1. Method of preparing a non-toxic reagent for the determination by haemagglutination of antibodies to bacterial toxins, characterised in that erythrocytes and the toxoid corresponding to the bacterial toxin are coupled by means of glutaraldehyde, by reacting glutaraldehyde with the erythrocytes in an aqueous buffer medium and then adding the bacterial toxin directly to the reaction medium.

2. Method of preparing a reagent for the determination by haemagglutination of antibodies to bacterial toxins, wherein the product obtained according to Claim 1 is further treated with a reagent for blocking aldehyde groups.

3. Method according to one of Claims 1 and 2, characterised in that
   a) glutaraldehyde is reacted with a suspension of erythrocytes in a buffered isotonic medium of pH 6.5 to 8 for a few minutes at a temperature of between 0° C and 37° C, such that the erythrocytes are present in the proportion of 0.75 to 8% (v/v) for a glutaraldehyde concentration of between 0.001 M and 0.1 M.
   b) toxin is reacted with the erythrocytes thereby obtained, in buffered isotonic suspension, for at least one hour in the presence of glutaraldehyde, in the proportion of 0.4 mg to 2 mg of toxin per ml of erythrocytes.
   c) the cells thus treated are washed to remove the excess reagents.

4. Method according to one of Claims 2 and 3, characterised in that the coupled cells are further treated, after centrifugation for at least 15 minutes, with an equal volume of a buffered aqueous solution of glycine or lysine having a final concentration of between 0.1 M and 1 M.

5. Method according to any one of the preceding Claims, characterised in that it relates to the preparation of a reagent for the determination of antitetanus antibodies.

6. Method according to any one of Claims 1 to 4, characterised in that it relates to the preparation of a reagent for the determination of antidiphtheria antibodies.

7. Non-toxic reagent for the determination by haemagglutination of antibodies to bacterial toxins, consisting of erythrocytes to which the toxoid corresponding to the bacterial toxin is coupled through the agency of glutaraldehyde, the structure of the said toxin being modified in such a way that it loses its toxic activity towards man or animals without denaturation of its antigenic sites.

8. Reagent according to Claim 7, characterised in that it is further stabilised by treatment with an agent for blocking the glutaraldehyde.

9. Reagent according to one of Claims 7 and 8, for the determination of antitetanus antibodies.

10. Reagent according to one of Claims 7 and 8 for the determination of antidiphtheria antibodies.

11. Method for determining antibodies to bacterial toxins, characterised in that the sample to be studied is mixed at different dilutions, in an aqueous buffer solution, with a reagent according to any one of Claims 7 to 10, in that there are observed the dilutions at which haemagglutination occurs and in that the concentration of antitoxin in the sample is determined by reference to a standard.

12. Method according to Claim 11, characterised in that the sample to be assayed is whole blood.

13. Method according to one of Claims 11 and 12, for the determination of antitetanus antibodies.

14. Method according to one of Claims 11 or 12, for the determination of antidiphtheria antibodies, characterised in that human serum albumin or a similar component is introduced into the determination medium.

15. Reagent kit for the determination by haemagglutination of antibodies to bacterial toxins, characterised in that it comprises a reagent according to any one of Claims 7 to 10, in the form of a buffered aqueous suspension containing coupled erythrocytes and a preservative, a buffered aqueous solution and a reference serum.

**Patentansprüche**

1. Verfahren zur Herstellung eines nicht-toxischen Reagens zur Bestimmung von Antikörpern, die gegen Bakterientoxine gerichtet sind, durch Hämagglutination, dadurch gekennzeichnet, daß die Erythrocyten und das dem Bakterientoxin entsprechende Toxoid mit Hilfe von Glutaraldehyd gekuppelt werden, wobei der Glutaraldehyd in gepuffertem wäßrigem Medium mit den Erythrocyten zur Reaktion gebracht wird, und dann das Bakterientoxin direkt dem Reaktionsmedium zugesetzt wird.

2. Verfahren zur Herstellung eines Reagens zur Bestimmung von Antikörpern, die gegen Bakterientoxine gerichtet sind, durch Hämagglutination, wobei das nach Anspruch 1 erhaltene Produkt außerdem mit einem Reagens zur Blockierung der Aldehyd-Funktionen behandelt wird.

3. Verfahren nach einem der Ansprüche 1 oder 2, dadurch gekennzeichnet, daß
   a) Glutaraldehyd mit einer Erythrocyten-Suspension einige Minuten lang bei einer Temperatur zwischen 0° C und 37° C in einem isotonischen, auf pH 6,5 bis 8 gepufferten Medium zur Reaktion gebracht wird, wobei die Erythrocyten mit einem Volumenverhältnis von 0,75 bis 8% vorhanden sind, bei einer Glutaraldehyd-Konzentration zwischen 0,001 M und 0,1 M;
   b) die so erhaltenen Erythrocyten in isotonischer gepufferter Suspension wenigstens eine Stunde lang in Gegenwart von Glutaraldehyd in einem Verhältnis von 0,4 mg bis 2 mg Toxin pro ml Erythrocyten zur Reaktion gebracht werden;
   c) die so behandelten Zellen zur Entfernung überschüssiger Reagenzien gewaschen werden.

4. Verfahren nach einem der Ansprüche 2 oder 3, dadurch gekennzeichnet, daß die gekuppelten Zellen nach Zentrifugation des weiteren wenigstens 15 Minuten lang mit einem gleichen Volumen einer wäßrigen gepufferten Glycocoll- oder Lysin-Lösung mit einer Endkonzentration zwischen 0,1 M und 1 M behandelt werden.

5. Verfahren nach irgendeinem der vorstehenden Ansprüche, dadurch gekennzeichnet, daß es die Herstellung eines Reagens zur Bestimmung von Antitetanus-Antikörpern betrifft.

6. Verfahren nach irgendeinem der Ansprüche 1 bis 4, dadurch gekennzeichnet, daß es die Herstellung eines Reagens zur Bestimmung von Antidiphtherie-Antikörpern betrifft.

7. Nicht-toxisches Reagens zur Bestimmung von Antikörpern, die gegen Bakterientoxine gerichtet sind, durch Hämagglutination, bestehend aus Erythrocyten, an die das dem Bakterientoxin entsprechende Toxoid über Glutaraldehyd gekuppelt ist, wobei die Struktur des Toxins in einer Weise modifiziert ist, daß es seine toxische Wirkung für Menschen und Tiere verliert, ohne Denaturierung seiner Antigen-Bereiche.

8. Reagens nach Anspruch 7, dadurch gekennzeichnet, daß es außerdem durch Behandeln mit einem Reagens zur Blockierung des Glutaraldehyds stabilisiert ist.

9. Reagens nach einem der Ansprüche 7 oder 8 zur Bestimmung von Antitetanus-Antikörpern.

10. Reagens nach einem der Ansprüche 7 oder 8 zur Bestimmung von Antidiphtherie-Antikörpern.

11. Verfahren zur Bestimmung von Antikörpern, die gegen Bakterientoxine gerichtet sind, dadurch gekennzeichnet, daß in einer gepufferten wäßrigen Lösung die zu untersuchende Probe in verschiedenen Verdünnungen mit einem Reagens nach irgendeinem der Ansprüche 7 bis 10 vermischt wird, daß beobachtet wird, bei welcher der Verdünnungen Hämagglutination eintritt, und daß die Antitoxin-Konzentration der Probe durch Vergleich mit einem Standard bestimmt wird.

12. Verfahren nach Anspruch 11, dadurch gekennzeichnet, daß die zu bestimmende Probe Vollblut ist.

**13.** Verfahren nach einem der Ansprüche 11 oder 12 zur Bestimmung von Antitetanus-Antikörpern.

**14.** Verfahren nach einem der Ansprüche 11 oder 12 zur Bestimmung von Antidiphtherie-Antikörpern, dadurch gekennzeichnet, daß in das Analysenmedium Human-Albuminserum oder eine analoge Verbindung eingebracht wird.

**15.** Reagenzienkoffer zur Bestimmung von Antikörpern, die gegen Bakterientoxine gerichtet sind, durch Hämagglutination, dadurch gekennzeichnet, daß er ein Reagens nach irgendeinem der Ansprüche 7 bis 10 in Form einer wäßrigen gepufferten Suspension - die gekuppelte Erythrocyten und ein Konservierungsmittel enthält - umfaßt, eine wäßrige Pufferlösung und ein Vergleichsserum.